(19) Europäisches Patentamt
European Patent Office
Office européen des brevets

(11) Publication number: **0 153 095**
**A1**

(12) # EUROPEAN PATENT APPLICATION

(21) Application number: **85300815.9**

(22) Date of filing: **07.02.85**

(51) Int. Cl.⁴: **C 07 H 3/08**
// C07H11/00, C07D475/04

(30) Priority: **14.02.84 JP 25699/84**

(43) Date of publication of application: **28.08.85**
**Bulletin 85/35**

(84) Designated Contracting States: **AT BE CH DE FR GB IT LI LU NL SE**

(71) Applicant: **Suntory Limited, 1-40 Dojimahama 2-chome Kita-ku, Osaka-shi Osaka-fu 530 (JP)**
Applicant: **SHIRATORI PHARMACEUTICAL CO. LTD., 6-11-24 Tsudanuma, Narashino-shi Chiba-ken (JP)**

(72) Inventor: **Sakai, Hideaki, 53 Iwai-cho Hogodaya-ku, Yokohama-shi Kanagawa-ken (JP)**

(74) Representative: **Ford, Michael Frederick et al, MEWBURN ELLIS & CO. 2/3 Cursitor Street, London EC4A 1BQ (GB)**

(54) **A process for procuding 5-doxy-L-arabinose.**

(57) 5-Deoxy-L-arabinose (IV) is produced in high yield and at low cost by oxidizing L-rhamnose dialkylmercaptal (I) with hydrogen peroxide in an acetic acid solvent, followed by decomposing the resulting oxidationproducts with ammonia.

The 5-deoxy-L-arabinose produced is useful as a starting material for synthesizing L-erythro-5,6,7,8-tetrahydrobiopterin.

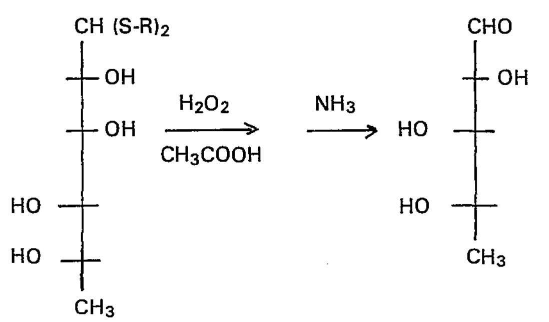


(I) (IV)

EP 0 153 095 A1

# A PROCESS FOR PRODUCING 5-DEOXY-L-ARABINOSE

## TECHNICAL FIELD

The present invention relates to a new process for producing 5-deoxy-L-arabinose. More specifically, it relates to a process for industrially or commercially producing 5-deoxy-L-arabinose from L-rhamnose dialkylmercaptal at a high yield and at a low cost.

## BACKGROUND ART

5-Deoxy-L-arabinose is a known compound useful as an intermediate for synthesizing L-erythro-5,6,7,8-tetrahydrobiopterin, which is expected to be effective and is now eagerly studied as a remedy for phenylketonuria (see J. Am. Chem. Soc., 78, page 5868, 1956).

5-Deoxy-L-arabinose must be synthesized because it is not naturally occurring sugar. 5-Deoxy-L-arabinose is conventionally produced by reacting L-rhamnose with ethyl mercaptan to form L-rhamnose diethylmercaptal, oxidizing the resultant L-rhamnose diethylmercaptal to 1,1-diethylsulfonyl-L-manno-2,3,4,5-tetrahydroxyhexane, then decomposing it with ammonia. This conventional production process, however, is disadvantageous in that the desired 5-deoxy-L-arabinose cannot be satisfactorily produced from the practical point of view. Various proposals have been made to use various oxidizing agents in the oxidation step of L-rhamnose diethylmercaptal. For example, processes using (i) propionic acid peroxide (see J. Chem. Soc. page 3544, 1955), (ii) sugar derivatives of phthalic acid peroxide (see J. Am. Chem. Soc., 78, pages 5868, 1956), and (iii) m-chloro perbenzoic acid (J. Am. Chem. Soc., 98, page 2301, 1976) have been proposed. However, the above-mentioned processes (i) and (ii) are not industrially satisfactory due to the fact that the yields of the desired product are as low as 49% and 35%, respectively. Furthermore, the above-mentioned process (iii) is disadvantageous in that the m-chloroperbenzoic acid is expensive, although the

yield of the desired product is acceptable.

DISCLOSURE OF THE INVENTION

Accordingly, an object of the present invention is to eliminate the above-mentioned disadvantages of the prior arts and to provide a process for producing 5-deoxy-L-arabinose at a high yield and at a low cost.

Another object of the present invention is to produce 5-deoxy-L-arabinose at a high yield by using, as an easily available and cheap oxidizing agent, hydrogen peroxide.

Other objects and advantages of the present invention will be apparent from the following descriptions.

In accordance with the present invention, there is provided a process for producing 5-deoxy-L-arabinose comprising the steps of (i) oxidizing L-rhamnose dialkylmercaptal with hydrogen peroxide in an acetic acid solvent and (ii) decomposing the resultant oxidation product with ammonia.

DETAILED DESCRIPTION OF WAY OF CARRYING OUT THE INVENTION

The present inventors studied to oxidize L-rhamnose dialkylmercaptal with a conventional oxidizing agent, hydrogen peroxide, which is commercially available at a low cost. When L-rhamnose dialkylmercaptal is oxidized with hydrogen peroxide under conventional conditions, however, the desired 1,1-diethylsulfonyl-L-manno-2,3,4,5-tetrahydroxyhexane cannot be substantially obtained. As a result of further study, the present inventors found that L-rhamnose dialkylmercaptal is easily oxidized and the desired 1,1-diethylsulfonyl-L-manno-2,3,4,5-tetrahydroxyhexane can be obtained at a high yield when a relatively large amount of acetic acid is used as a solvent.

Thus, according to the present invention, L-rhamnose dialkylmercaptal (I) is oxidized with hydrogen peroxide in acetic acid and the resultant oxidation product (II)

0153095

and (III) are decomposed with ammonia to produce the desired 5-deoxy-L-arabinose (IV), as follows:

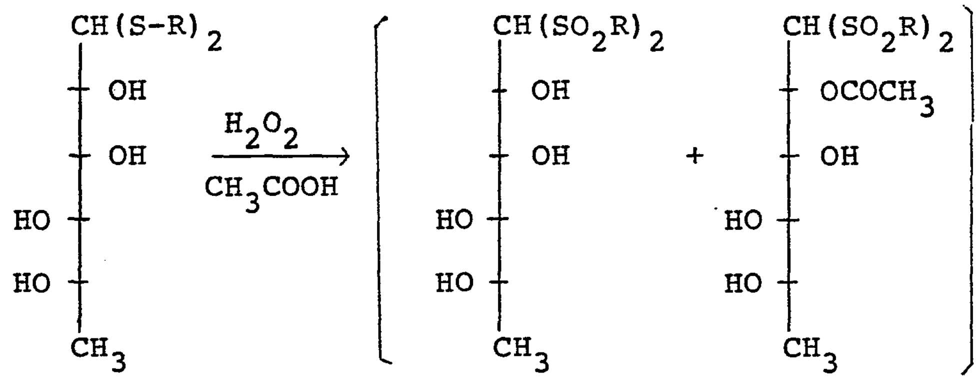


(I) (II) (III)

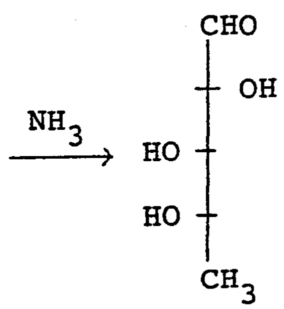


(IV)

wherein R represents a lower alkyl group, preferably an alkyl group having 1 to 3 carbon atoms.

In the practice of the preferred embodiment of the present invention, L-rhamnose dialkylmercaptal (I) is first suspended or dissolved in acetic acid. The oxidation reaction is then carried out by adding hydrogen peroxide to the resultant suspension or solution. In this oxidation reaction, the amount of the acetic acid used as a solvent affects the oxidation reaction. The use of too small an amount of the acetic acid tends to result in insufficient progress of the desired oxidation reaction and the generation of undesirable side reactions. Accordingly, the acetic acid solvent is generally used in an amount of at least 3 times by weight, preferably 4 to 10 times by weight, and more preferably 5 to 6 times by weight, of the amount of the L-rhamnose dialkylmercaptal (I) in the reaction mixture.

The use of too large an amount of the acetic acid is not preferable, because it is not economical since the acetic acid should be removed from the reaction mixture.

Although hydrogen peroxide is theoretically used in an amount 4 times by weight of the amount of the L-rhamnose dialkylmercaptal (I), the hydrogen peroxide is practically and preferably used in a slightly excess amount, e.g., in an amount 4.3 to 5 times by weight of the amount of the L-rhamnose dialkylmercaptal.

The oxidation reaction can be carried out by, for example, dropwise adding the hydrogen peroxide to the reaction mixture at a temperature of 30°C or less, followed by reacting at a temperature of 20°C to 30°C for 10 to 50 hours.

The resultant oxidation reaction mixture obtained above is preferably subjected to, for example, a distillation and concentration step, whereby the excess hydrogen peroxide and the acetic acid solvent are removed therefrom. The resultant residue is then decomposed with ammonia.

However, in the preferred embodiment of the present invention, the above-mentioned oxidation reaction mixture is first treated with a sulfite or bisulfite to decompose the excess hydrogen peroxide before the removal of the excess hydrogen peroxide and the acetic acid solvent. This is because, when the oxidation reaction mixture is directly concentrated on a large industrial scale, the resultant oxidation product is unpreferably decomposed to remarkably decrease the yield of the desired 5-deoxy-L-arabinose. This undesirable decomposition of the resultant oxidation product can be effectively prevented when the concentration of the oxidation reaction mixture is carried out in the presence of the sulfite or bisulfite.

Examples of the sulfites or bisulfites usable in the present invention are sulfites such as lithium sulfite, sodium sulfite, potassium sulfite, and ammonium sulfite

and bisulfites such as sodium bisulfite, potassium bisulfite, and ammonium bisulfite. Although there is no critical amount of the sulfite or bisulfite, the sulfite or bisulfite is preferably used in an amount of at least 2 times by mole, preferably 2 to 4 times by mole of the amount of the excess hydrogen peroxide.

The concentrated residue of the oxidation reaction mixture obtained above contains a mixture of 1,1-dialkylsulfonyl-L-manno-2,3,4,5-tetrahydroxyhexane (II) and 1,1-dialkylsulfonyl-L-manno-monoacetyloxy-3,4,5-trihydroxyhexane (III). This mixture can be directly decomposed, without separation, with ammonia in the subsequent step. The compounds (II) and (III) can be decomposed with ammonia in any conventional manner. For example, a mixture of the compounds (II) and (III) is the resultant residue may be first dissolved in water and ammonia then added to the resultant aqueous solution to adjust the pH of the aqueous solution to 9 to 10. The compounds (II) and (III) are thus treated with ammonia for 10 to 20 hours at room temperature. As a result, the desired 5-deoxy-L-arabinose (IV) can be obtained at a high yield.

The 5-deoxy-L-arabinose (IV) thus produced can be converted to L-erythro-5,6,7,8-tetrahydrobiopterin (VI) as follows.

5-Deoxy-L-arabinose + phenylhydrazine

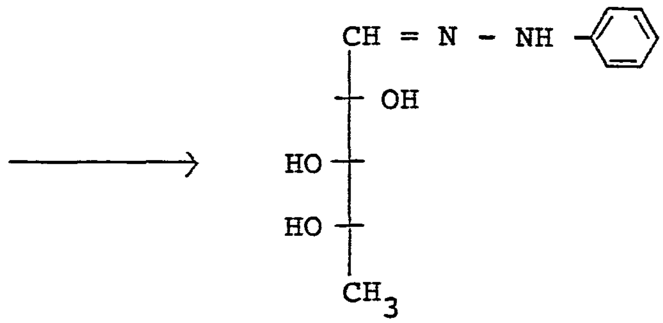

(V)

(V) +

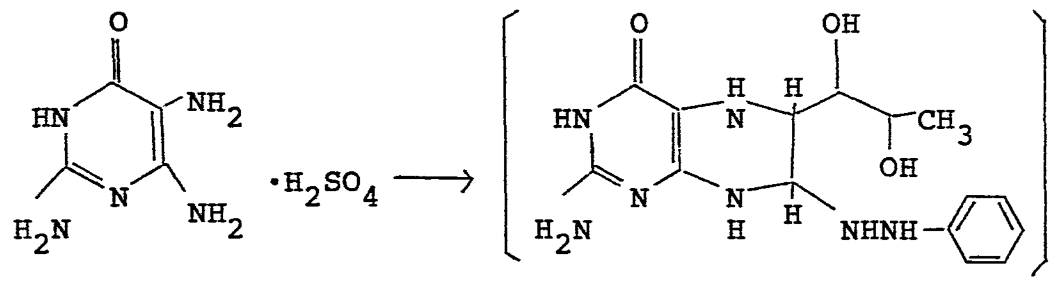

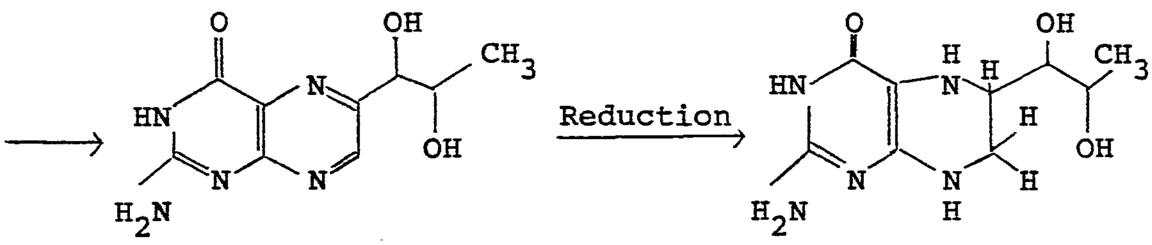


(VI)

L-Biopterin Tetrahydro-L-biopterin

These reactions are disclosed in, for example, Bull. Chem. Soc. Japan, 48, page 3767, 1975 et al, J. Am. Chem. Soc., 78, page 5868, 1956 ibid, 98, page 2301, 1976, J. Chem. Soc(C)., 1969, page 928, and Helv. Chim. Acta, 52, page 1225, 1969. As mentioned hereinabove, the resultant tetrahydro-L-biopterin is effective for curing phenylketonuria as disclosed in, for example, Lancet, 1979, page 131 and N. Engl. J. Med., 293, page 785, 1975.

The present invention now will be further illustrated by, but is by no means limited to, the following examples.

Example 1

A 5.4 g amount of L-rhamnose diethylmercaptal was suspended in 30 ml of acetic acid. Then, 9.3 g of a 30%

aqueous hydrogen peroxide was dropwise added to the resultant suspension at a temperature of 20°C to 30°C over 10 minutes. After the completion of the dropwise addition, the reaction mixture was allowed to stand while stirring at the same temperatures for 15 hours.

A 40 ml amount of water was then added to the resultant oxidation reaction mixture and the resultant mixture was concentrated in vacuo at a bath temperature of 45°C or less. Thus, 7.7 g of a mixture of 1,1-diethylsulfonyl-L-manno-2,3,4,5-tetrahydroxyhexane and 1,1-diethylsulfonyl-L-manno-monoacetyloxy-3,4,5-trihydroxyhexane was obtained.

Thereafter, 7.7 g of the resultant mixture obtained above was dissolved in 25 ml of water, and the pH of the resultant aqueous solution was precisely adjusted to 9.2 by gradually adding a 28% aqueous ammonia. The mixture was allowed to stand while stirring at room temperature for 15 hours. The precipitated diethylsulfonyl methane was removed by filtration and the filtrate was treated by an ion exchange resin (i.e., Lewatit S-100, 70 ml + Lewatit MP-62, 70 ml, both resins available from Bayer A.G.).

The solution thus treated was concentrated is vacuo. The remaining diethylsulfonyl methane was removed by being extracted with chloroform. The resultant aqueous phase was distillated off in vacuo. Thus, 2.0 g (yield = 75%) of 5-deoxy-L-arabinose was obtained in the form of pale yellow oil.

The infrared spectrum (IR), nuclear magnetic resonance spectrum (NMR), optical rotation, and thin layer chromatography (TLC) ($CHCl_3:C_2H_5OH:CH_3COOH$ = 10:7:3) analysis data of the product obtained above were identical to those of the authentic sample.

The IR and NMR data were as follows:

IR (film, $cm^{-1}$): 3350-3380, 2930-2980, 1650, 1125, 1065, 1035, 990

NMR($D_2O$, TSP)$\delta$: 1.38(d), 3.54-4.40(m), 5.16(t)

Example 2

A 500 g amount of L-rhamnose diethylmercaptal was suspended in 2.75 liters of acetic acid. Then, 923.5 g of a 30% aqueous hydrogen peroxide was dropwise added to the resultant suspension at a temperature of 20°C to 30°C over 90 minutes. After the completion of the dropwise addition, the reaction mixture was allowed to stand while stirring at the same temperature for 45 hours.

Thereafter, 283.6 g of sodium sulfite was dissolved in 1.4 liter of water in an ice bath and was dropwise added to the reaction mixture to remove the excess hydrogen peroxide therefrom.

The resultant mixture was concentrated in vacuo at a bath temperature of 45°C. Then, 1 liter of methanol was added to the concentrated mixture and the precipitated inorganic salts were removed by filtration. The filtrate was concentrated in vacuo to obtain 819.65 g of a mixture of 1,1-diethylsulfonyl-L-manno-2,3,4,5-tetrahydroxyhexane and 1,1-diethylsulfonyl-L-manno-3,4,5-trihydroxyhexane.

Then, 819.65 g of the mixture obtained above was dissolved in 2.3 liters of water, and 274 ml of a 28% aqueous ammonia was gradually added to the resultant solution to precisely adjust the pH of the solution to 9.2. The mixture was allowed to stand while stirring at room temperature for 15 hours. The precipitated diethylsulfonyl methane was removed by filtration and the filtrate was treated with an ion exchange resin (i.e., Lewatit S-100, 8 liters + Lewatit MP-62, 8 liters).

The solution thus treated was concentrated in vacuo. The remaining diethylsulfonyl methane was removed by being extracted with chloroform. The resultant aqueous phase was distilled off in vacuo. Thus, 213 g (yield = 84%) of the desired 5-deoxy-L-arabinose was obtained in the form of pale yellow oil.

The IR, NMR, optical rotation, and TLC ($CHCl_3$:

$C_2H_5OH:CH_3COOH$ = 10:7:3) analysis data of the product obtained above were identical to those of the authentic sample, as in Example 1.

CLAIMS

1. A process for producing 5-deoxy-L-arabinose characterized by comprising the steps of:

(i) oxidizing L-rhamnose dialkylmercaptal with hydrogen peroxide in an acetic acid solvent and

(ii) decomposing the resultant oxidation product with ammonia.

2. A process as claimed in claim 1, wherein a sulfite or bisulfite is added to the oxidation reaction mixture and, after removing the acetic acid therefrom, the resultant residue is treated with the ammonia.

3. A process as claimed in claim 1 or 2, wherein the acetic acid is used in an amount of at least 3 times by weight of the amount of the L-rhamnose dialkylmercaptal.

European Patent Office

# EUROPEAN SEARCH REPORT

0153095
Application number

EP 85 30 0815

| Category | Citation of document with indication, where appropriate, of relevant passages | Relevant to claim | CLASSIFICATION OF THE APPLICATION (Int. Cl.4) |
|---|---|---|---|
| Y | JOURNAL OF THE AMERICAN CHEMICAL SOCIETY, vol. 74, no. 5, 5th March 1952, pages 1225-1230; MARSHALL W. CRONYN: "Sulfones. I. Methods for the preparation of certain alkanes, alkenes, acids and lactones with Bis-(ethylsulfonyl)-methane" * Page 1228, Bis-(ethylsulfonyl)-methane * --- | 1,3 | C 07 H 3/08 // C 07 H 11/00 C 07 D 475/04 |
| A | "Methoden der organischen Chemie", (Houben-Weyl), vol. VIII, Sauerstoffverbindungen III, 1952, pages 62,63,72, edited by Eugen Müller, Georg Thieme Verlag, Stuttgart, DE; R. CRIEGEE: "Herstellung und Umwandlung von Peroxyden" * Pages 62-63; page 72 * --- | 2 | |
| | | | TECHNICAL FIELDS SEARCHED (Int. Cl.4) |
| Y | US-A-3 505 329 (WEINSTOCK) * Column 2, line 25 - column 3, line 28 * --- | 1,3 | C 07 H |
| Y | L.F. FIESER et al.: "Reagents for organic synthesis", 1967, pages 457-465, John Wiley and Sons, Inc., New York, US; Pages 457-465: "Hydrogen peroxide, acidic"; pages 785-791: "Peracetic acid" * Pages 459-460,785,787 * --- -/- | 1 | |

The present search report has been drawn up for all claims

| Place of search | Date of completion of the search | Examiner |
|---|---|---|
| THE HAGUE | 10-04-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

**EUROPEAN SEARCH REPORT**

0153095
Application number

EP 85 30 0815



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF THE CHEMICAL SOCIETY, 1955, pages 3544-3548, London, GB; L. HOUGH et al.: "1: 1-Diethylsulphonyl derivatives of L-rhamnose and their conversion into 5-deoxy-L-arabinose" * Formulas I-V; page 3545; Experimental page 3547 * --- | 1,3 | |
| D,Y | JOURNAL OF THE AMERICAN CHEMICAL SOCIETY, vol. 98, no. 8, 14th April 1976, pages 2301-2307; E.C. TAYLOR et al.: "Pteridines. XXXVII. A total synthesis of L-erythro-biopterin and some related 6-(polyhydroxyalkyl)pterins" * Scheme I, page 2301; scheme IV, page 2303; experimental, page 2306 * --- | 1,3 | TECHNICAL FIELDS SEARCHED (Int. Cl.4) |
| Y | BERICHTE DER DEUTSCHEN CHEMISCHEN GESELLSCHAFT, vol. 69, 1936, pages 1610-1615, Verlag Chemie GmbH, Berlin, DE; H. BÖHME: "Zur Kenntnis der alpha-halogenierten Thioäther (I. Mitteil.) * Page 1614, "Methylen-bis-äthylsulfon" * ----- | 1,3 | |

The present search report has been drawn up for all claims

| Place of search | Date of completion of the search | Examiner |
|---|---|---|
| THE HAGUE | 10-04-1985 | VAN AMSTERDAM L.J.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document